# EUROPEAN PATENT APPLICATION

(11) **EP 4 417 215 A1**
(43) Date of publication of application: **21.08.2024**
(21) Application number: 23305219.0
(22) Date of filing: 20.02.2023
(51) Int. Cl.: A61K 38/20, A61P 37/08

(54) **INTERLEUKIN-1 FOR THE TREATMENT AND PREVENTION OF ALLERGIES**

(71) Applicant: SORBONNE UNIVERSITE, 75006 Paris (FR); Institut National de la Santé et de la Recherche Médicale, 75013 Paris (FR); Assistance Publique Hôpitaux de Paris, 75012 Paris 12 (FR)
(72) Inventor: KLATZMANN, David, 75013 PARIS (FR); ENGEROFF, Paul, 75013 PARIS (FR); BELLIER, Bertrand, 75013 PARIS (FR); GRAFF-DUBOIS, Stéphanie, 75013 PARIS (FR); BELBEZIER, Aude, 75013 PARIS (FR)
(74) Representative: Gevers & Orès

(57) **Abstract**

The invention pertains to interleukin-1 (IL-1) and compositions comprising the same for use in the prevention and/or the treatment of an allergy, particularly in a method of desensitization. The invention also pertains to compositions comprising IL-1 with an allergen, medical devices comprising the same and kits for desensitization.

## Description

The invention pertains to interleukin-1 (IL-1) and compositions comprising the same for use in the prevention and/or the treatment of an allergy, particularly in a method of desensitization. The invention also pertains to compositions comprising IL-1 with an allergen, medical devices comprising the same and kits for desensitization.

Allergies are one of the fastest growing conditions worldwide, becoming a major public health problem. They disrupt the lives of millions of people and can cause extremely serious complications. More than one in every four people in Europe currently suffer from allergies, and the World Health Organization (WHO) predicts that by 2050 one in two people in the world will be affected, especially in industrialized countries.

Allergies are caused by a deregulation of the immune system, which overreacts to substances that are normally harmless. These substances, known as *"*allergens*"*, can be found in the air (pollen, cat hair, etc.), in food (milk, eggs, peanuts, etc.), in medicines (penicillin, curare, etc.) and in materials we come into contact with (latex, nickel, etc.). The allergies they trigger can lead to a variety of manifestations, such as dermatitis, eczema, rhinitis, asthma attacks, edema, or anaphylaxis.

The immediate manifestations of allergies are mainly due to type I hypersensitivity (also called immediate hypersensitivity). Type I hypersensitivity is a misguided immune response to a non-pathogenic antigen resulting in elevated levels of Immunoglobulin E (IgE). IgE sensitizes mast cells and basophils via the high-affinity IgE receptor FcεRI, gearing the cells up to de-granulate upon antigen encounter resulting in the release of highly inflammatory mediators. In type I hypersensitivity, IgG antibodies play a key role in regulating IgE activity in that they can either neutralize the allergen (anti-allergen IgG), neutralize IgE (anti-IgE IgG), or inhibit FcεRI signaling directly on mast cells and basophils by cross-linking FcεRI with the immunoreceptor tyrosine-based inhibitory motif (ITIM) containing IgG receptor FcyRllb. Both IgG and IgE responses are regulated by follicular T helper (TFH) and regulatory cells (TFR) controlling the germinal center B cell (GCB) reaction.

The only disease-modifying treatment for allergic patients is allergen-specific immunotherapy (also called desensitization), which is based on a repeated administration of the allergen at increasing doses, resulting in an increase of regulatory T and B cells as well as an elevated IgG:IgE ratio. Those hallmarks of allergen-specific immunotherapy are at the basis of novel therapeutic approaches to improve allergy immunotherapy.

However, about half of the people who receive desensitization experience mild side effects, such as mild rash, sneezing, watery eyes, mild asthma symptoms, itching, tiredness, and headaches. In very rare cases, immunotherapy can even cause an anaphylactic reaction. This can lead to severe problems like nausea, breathing difficulties, circulation problems. In the most severe cases, it may result in an anaphylactic shock, requiring immediate medical attention and an adrenaline (epinephrine) injection to quickly limit the dangerous overreaction occurring in the body. Moreover, the risk of side effects varies among allergens and thus limits the use of desensitization for all allergens, e.g., peanut or cat allergy immunotherapy is rarely done.

Thus, there remains a genuine need for new means to treat allergies and to reduce the side effects of desensitization methods for allergic individuals.

The present invention is believed to meet such need by providing a new use of interleukin 1 (IL-1) to prevent or treat allergies or reduce their side effects.

Using a mouse model based on Alum/Ovalbumin sensitization, the Inventors surprisingly found that addition of IL-1 during immunization confers protection from anaphylaxis by promoting the IgG/lgE ratio. Furthermore, IL-1 directly up-regulates FcyRllb on mast cells, reducing their reactivity upon allergen challenge. Also, the absence of IL-1R2 in TFR cells resulted in a significantly decreased IgG/lgE ratio and enhanced IL-1 dependent proliferation of TFR cells. In addition, the Inventors demonstrated the therapeutic potential of low-dose IL-1 in models of systemic anaphylaxis and food allergy. IL-1 orchestrates the suppression of allergic responses by regulating follicular T cells and mast cells and thus bears potential as an adjuvant for the improvement of safety as well as efficacy in allergy immunotherapy.

In an aspect, the invention thus relates to interleukin-1 (IL-1) for use in the prevention and/or the treatment of an allergy.

The present invention further relates to a composition comprising IL-1 as an active ingredient for use in the prevention and/or the treatment of an allergy.

The present invention further relates to a method for treating and/or preventing an allergy by means of administration, to an individual in need thereof, of IL-1 or a composition comprising IL-1.

The present invention further relates to the use of IL-1 or a composition comprising IL-1 for the manufacture of a topical medication for treating and/or preventing an allergy.

As used herein, the expression *"*interleukin-1*"* (or *"*IL-1*"*) designates interleukin-1α (IL-1α) and interleukin-1β (IL-1β). Both cytokines are agonists and are expressed in multiple cell lines including monocytes, macrophages, neutrophils, hepatocytes, and tissue macrophages throughout the body. Despite the fact that IL-1α and IL-1β differ in their amino acid sequence, both can be synthesized as a 33 kDa, 271 aa pro-cytokine that is enzymatically cleaved (by calpain) into a bio-active 17 kDa, 159 aa mature segment and a 112 aa pro-sequence.

In the invention, interleukin-1 can thus be IL-1α or IL-1β, preferably IL-1β.

In an embodiment, interleukin-1 is a mammal IL-1, preferably a human IL-1.

Within the context of the invention, *"*IL-1*"* also includes natural or artificial variants of IL-1 having at least about 90% amino acid sequence identity with the sequence of IL-1α or IL-1β. Variants can be generated using classic genetic engineering, e.g., by insertion, substitution, deletion, or a combination thereof. Substitutions in a protein sequence of the invention can be conservative or non-conservative.

Without limiting the invention, some reference sequences of IL-1α and IL-1β are given in Table 1.

**Table 1. Reference sequences of IL-1α and IL-1β.**

| | |
|---|---|
| **SEQ ID NO: 1** | MAKVPDMFEDLKNCYSENEEDSSSIDHLSLNQKSFYHVSYGPLHEGCMDQSVSLS ISETSKTSKLTFKESMVVVATNGKVLKKRRLSLSQSITDDDLEAIANDSEEEIIKPRSA PFSFLSNVKYNFMRIIKYEFILNDALNQSIIRANDQYLTAAALHNLDEAVKFDMGAY KSSKDDAKITVILRISKTQLYVTAQDEDQPVLLKEMPEIPKTITGSETNLLFFWETHG TKNYFTSVAHPNLFIATKQDYWVCLAGGPPSITDFQILENQA |
| Human IL-1α | |
| UNIPROT Accession No. P01583 | |
| **SEQ ID NO: 2** | MAEVPELASEMMAYYSGNEDDLFFEADGPKQMKCSFQDLDLCPLDGGIQLRISD HHYSKGFRQAASVVVAMDKLRKMLVPCPQTFQENDLSTFFPFIFEEEPIFFDTWD NEAYVHDAPVRSLNCTLRDSQQKSLVMSGPYELKALHLQGQDMEQQVVFSMSF VQGEESNDKIPVALGLKEKNLYLSCVLKDDKPTLQLESVDPKNYPKKKMEKRFVFN KIEINNKLEFESAQFPNWYISTSQAENMPVFLGGTKGGQDITDFTMQFVSS |
| Human IL-1β | |
| UNIPROT Accession No. P01584 | |

In an embodiment, interleukin-1 has an amino acid sequence having at least 90% sequence identity with SEQ ID NO: 1 or SEQ ID NO: 2.

In an embodiment, interleukin-1 has an amino acid sequence having at least 91%, 92%, 93%, 94%, 95%, 96%, 97%, 98%, 99% or 100% sequence identity with SEQ ID NO: 1.

In an embodiment, interleukin-1 has an amino acid sequence having at least 91%, 92%, 93%, 94%, 95%, 96%, 97%, 98%, 99% or 100% sequence identity with SEQ ID NO: 2.

In an embodiment, interleukin-1 has an amino acid sequence having at least 95 %, preferably at least 99%, more preferably 100% identity with SEQ ID NO: 1.

In an embodiment, interleukin-1 has an amino acid sequence having at least 95 %, preferably at least 99%, more preferably 100% identity with SEQ ID NO: 2.

"Identity" with respect to percent amino acid sequence "identity" is defined herein as the percentage of amino acid residues in the candidate sequence that are identical with the residues in the target sequences after aligning both sequences and introducing gaps, if necessary, to achieve the maximum percent sequence identity. Percent sequence identity is determined by conventional methods. Briefly, for example, two amino acid sequences can be aligned to optimize the alignment scores using the ClustalW algorithm (Thompson et al., Nuc. Ac. Res. 22:4673-4680, 1994) and PAM250 weight matrix (Dayhoff et al., "Atlas of Protein Sequence and Structure." National Biomedical Research Foundation. Washington, DC 5:345-358, 1978) and default parameters as provided by the program MegAlign (DNASTAR, Inc.; Madison, Wl). The percent identity is then calculated as: [Total number of identical matches x 100] divided by [length of the longer sequence + number of gaps introduced into the longer sequence in order to align the two sequences].

In an embodiment, interleukin-1 is fused to another protein, in particular a carrier protein and/or another biologically active protein.

In the present invention, the term "allergy" designates any allergy caused by any allergen, preferably an allergy that is associated to a type I hypersensitivity.

In an embodiment, the allergy is caused by an auto-allergen.

As used herein, the expression *"*auto-allergen*"* designates a self-antigen that drives inflammation through IgE.

In an embodiment, the auto-allergen is a double stranded DNA (dsDNA). In particular, dsDNA is associated to systemic lupus erythematosus.

In an embodiment, the auto-allergen is IL-24. In particular, IL-24 is associated to chronic spontaneous urticaria.

In an embodiment, the invention relates to IL-1 or a composition comprising IL-1 for use as defined above, wherein the IL-1 or the composition comprising IL-1 is used in a method of desensitization of an allergic individual.

According to the invention, the individual is an animal, preferably a human.

As used herein, the expression *"*method of desensitization*"* (or desensitization) refers to a well-known method for prevention or reduction of immediate hypersensitivity by administration of graded doses of an allergen.

As used herein, the term *"*allergen*"* refers to a molecule that can induce an allergic reaction and to any substance, composition or material comprising such a molecule. The allergen may be of various nature, such as a lipid, protein, peptide, polypeptide, chemical compound, metal, plastic, etc. The allergen may be in a natural state or produced artificially (e.g., by recombinant and/or enzymatic techniques for instance). The allergen may be structurally altered or modified to improve its stability, immunogenicity, etc. The allergen may be a mixture of several molecules (e.g., an extract). The allergen can be used in different states, such as liquid or dry.

In an embodiment, the invention relates to IL-1 or a composition comprising IL-1 for use as defined above, wherein IL-1 is used in combination with at least an allergen.

As used herein, the expression "IL-1 is used in combination with at least an allergen" indicates that IL-1 and at least one allergen are administered to an individual, at the same time or in different times. IL-1 and said at least one allergen can be present in the same composition or in different compositions.

In an embodiment, the invention relates to a composition comprising IL-1 for use as defined above, wherein said composition further comprises at least an allergen.

In an embodiment, the invention relates to a composition comprising IL-1 for use as defined above, wherein said allergen is a food, contact or respiratory allergen or a drug.

As used herein, the expression *"*food allergen*"* refers to any allergen that can be found in food, such as, without limitation, groundnut, peanut, milk, egg, tree nuts and seeds (such as but not limited to: hazelnut, cashew, walnut, pecan, Brazil nut, macadamia, chestnut, pistachio, coconut, almond, sesame, mustard), fish, shellfish, crustaceans, cereals (such as but not limited to: wheat, corn, oat, barley, rye, rice, sorghum, spelt), legumes (such as but not limited to: soy, kidney bean, black bean, common bean, chickpea, pea, cow pea, lentils, lupine) or mixtures thereof.

As used herein, the expression *"*contact allergen*"* refers to any allergen that can be in contact with the skin of an individual, such as, without limitation, latex, nickel, gold, fragrance mixes, thimerosal, neomycin sulphate, formaldehyde, cobalt chloride, bacitracin, Quaternium 15 or mixtures thereof.

As used herein, the expression *"*respiratory allergen*"* refers to any allergen that can be present in the air, such as, without limitation, dust mites, rye, ragweed, cockroaches, pollen, mold, animal dander, animal hair, dust or mixtures thereof.

As used herein, the expression *"*drug*"* refers to any medicine that can induce an allergic response, such as, without limitation, an antibiotic (e.g., penicillin), a pain reliever (e.g., aspirin, ibuprofen and naproxen sodium), chemotherapy drugs, venoms or drugs for autoimmune diseases or mixtures thereof.

In an embodiment, the allergen is egg or a compound found in egg, in particular the ovalbumin.

In an embodiment, the invention relates to IL-1 or a composition comprising IL-1 for use as defined above, wherein the IL-1 or the composition comprising IL-1 is used in a method of immunotherapy. As used herein, the term *"*immunotherapy*"* designates the administration of a medicine to manipulate the immune system of an individual. Immunotherapy includes, but is not limited to, desensitization, vaccination, some treatments of cancer cells or some treatments of immune diseases. Immunotherapy can be either active or passive. In active immunotherapy, the individual is administered with a molecule that sets in motion an immune response against this molecule, e.g., vaccination against a pathogen, desensitization to an allergen or administration of a tumor antigen. In passive immunotherapy, immune molecules are given to patients who do not produce them on their own, e.g., administration of antibodies specific to a pathogen or a tumor. In the present invention, the administration of IL-1 is useful to prevent or treat an allergy that may occur during an immunotherapy, or at least reducing its side effects.

In an embodiment, the invention relates to IL-1 or a composition comprising IL-1 for the treatment or the prevention of anaphylaxis, in particular of an anaphylactic shock.

In an embodiment, the invention relates to IL-1 or a composition comprising IL-1 for use as defined above, said composition being administered by oral route, epicutaneous route, subcutaneous route, transdermal route, intralymphatic route, intramuscular route, intravenous route, nasal route, or rectal route.

In another aspect, the invention also relates to a composition comprising IL-1 and at least an allergen.

In an embodiment, the invention relates to a composition as defined above, said composition being in the form of a solution, a gel, a powder, an aerosol spray, a lotion, or a foam.

In an embodiment, the invention relates to a composition as defined above, wherein said allergen is a food, contact or respiratory allergen or a drug.

In another aspect, the invention also relates to a medical device comprising a composition comprising IL-1 and at least an allergen, said medical device being appropriate for the administration of said composition to an individual.

In an embodiment, the invention relates a medical device as defined above, said medical device being selected from the group comprising a patch, an inhaler, a nebulizer, and a syringe.

In another aspect, the invention relates a kit for desensitization comprising:
- a composition comprising IL-1, and
- at least an allergen.

The following figures and examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

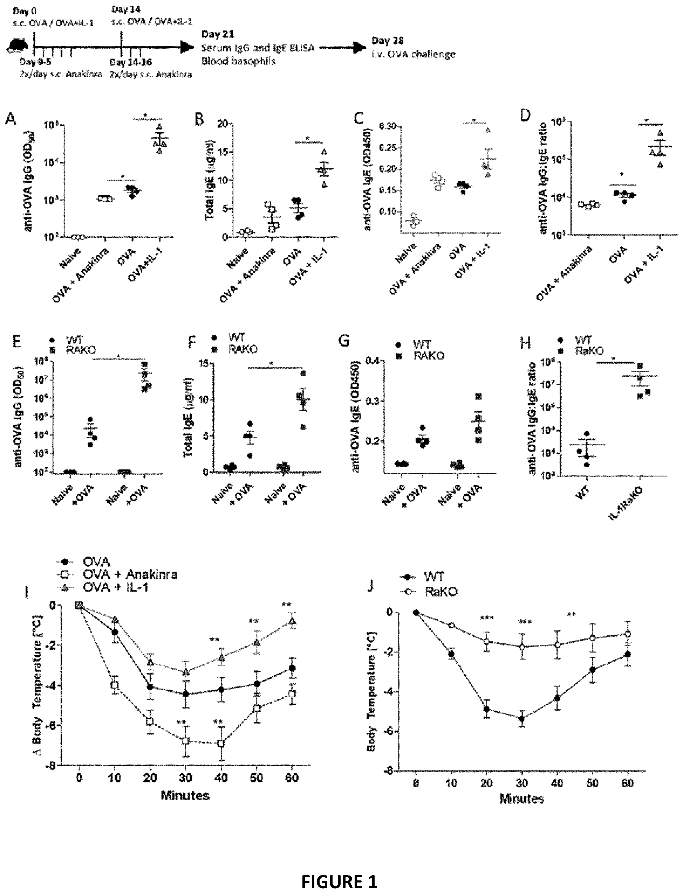

**Figure 1****.** Mice were immunized with OVA, OVA+IL-1 or treated with Anakinra (OVA+Anakinra), serum was collected at day 0 and day 21. **A.** Shown are mean ± SEM OD50 titers of OVA-specific IgG. **B.** Mean ± SEM total IgE levels (µg/ml). **C.** Mean ± SEM OD450 values of OVA-specific IgE. **D.** Specific IgG to specific IgE ratio. **E.** Wild type or IL-1RaKO mice were immunized with OVA. Shown are mean ± SEM OD50 titers of OVA-specific IgG. **F.** Mean ± SEM total IgE levels (µg/ml). **G.** Mean ± SEM OD450 values of OVA-specific IgE. **H.** Specific IgG to specific IgE ratio. **I.** At day 28, mice were challenged i.v. with OVA and body temperature was measured with a 10-minute interval between each measure. Shown are mean ± SEM degrees lost compared to baseline temperature in OVA, OVA+IL-1, or OVA+Anakinra mice. J. Shown are mean ± SEM degrees lost compared to baseline temperature in Wild type or IL-1RaKO mice.

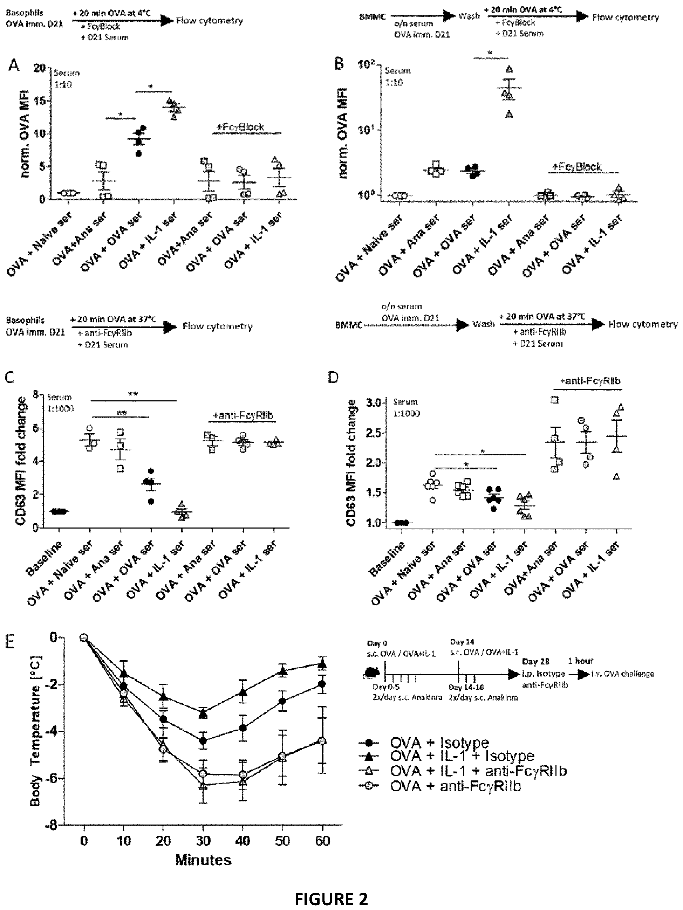

**Figure 2****.** The impact of D21 immunized serum on OVA binding to effector cells is shown as the mean ± SEM relative OVA-A488 MFI fold change compared to naive serum. **A.** OVA-A488 binding to basophils in presence of 1:10 serum. **B.** OVA-A488 binding to BMMCs in presence of 1:10 serum. **C.** The impact of D21 immunized serum on effector cell degranulation is shown as the mean ± SEM anti-CD63 MFI fold change relative to non-challenged cells (baseline). **D.** Serum inhibition assay with basophils in presence of 1:1000 serum. **D.** Serum inhibition assay with sensitized BMMCs in presence of 1:1000 serum. **E.** OVA challenge at day 28 in presence of anti-FcyRllb blocking antibody or isotype control antibody. Shown are mean ± SEM degrees lost compared to baseline temperature.

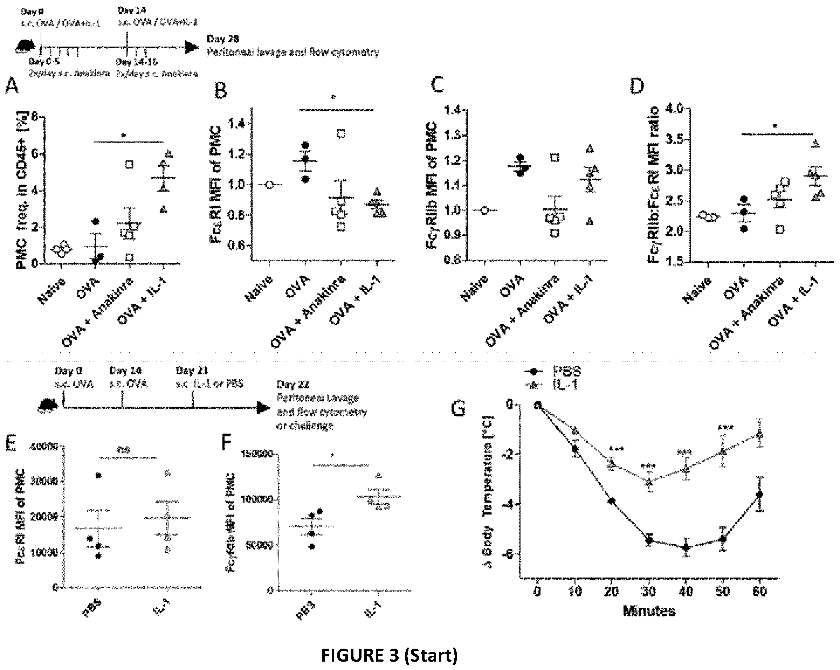

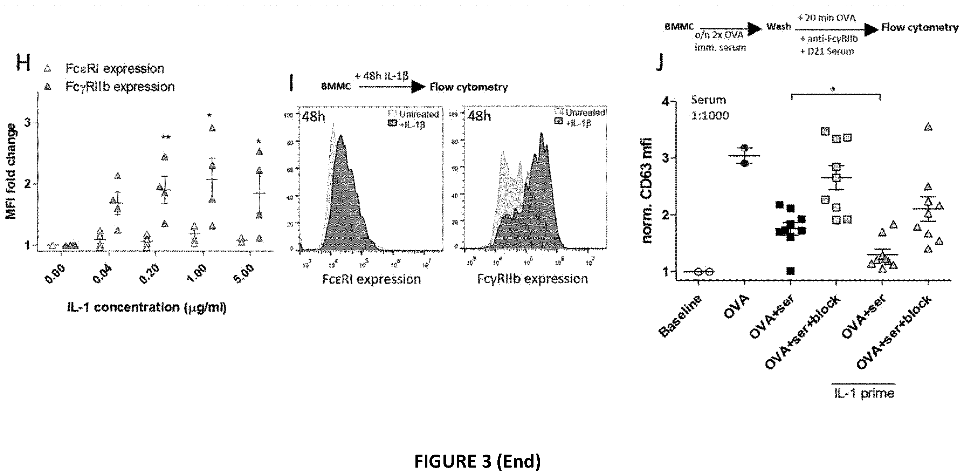

**Figure 3****.** Mice were immunized with OVA, OVA+IL-1, or OVA+Anakinra and PMCs were investigated at day 28. **A.** Shown are mean ± SEM PMC frequencies. **B.** Mean ± SEM anti-FcεRI MFl in PMC. **C.** Mean ± SEM FcyRllb MFI in PMC. **D.** FcγRIIb:FcεRI MFI ratio. **E.** OVA-immunized mice were injected with s.c. PBS or IL-1 at day 21. At day 22, PMC were investigated by flow cytometry. Shown is the mean ± SEM anti-FcεRI MFI in PMC. **F.** Shown is the mean ± SEM FcyRllb MFI in PMC. **G.** Alternatively, mice were challenged with OVA at day 22. Shown are mean ± SEM degrees lost compared to baseline temperature. **H.** BMMCs were treated with various concentrations of IL-1β for 48h. Shown are mean ± SEM anti-FcyRllb and anti-FcεRI MFI fold changes compared to unstimulated control cells. **I.** Representative BMMC raw histograms of anti-FcεRI and anti-FcyRllb expression in absence or presence of 0.2ug/ml IL-1β for 48h. **J.** Sensitized BMMCs were primed for 48h with 0.2ug/ml IL-1β. OVA was mixed with serum from D21 OVA immunized WT mice and added to IL-1β primed or non-primed BMMCs in additional presence or absence of anti-FcyRllb. Shown is the mean ± SEM relative anti-CD63 expression compared to untreated control cells.

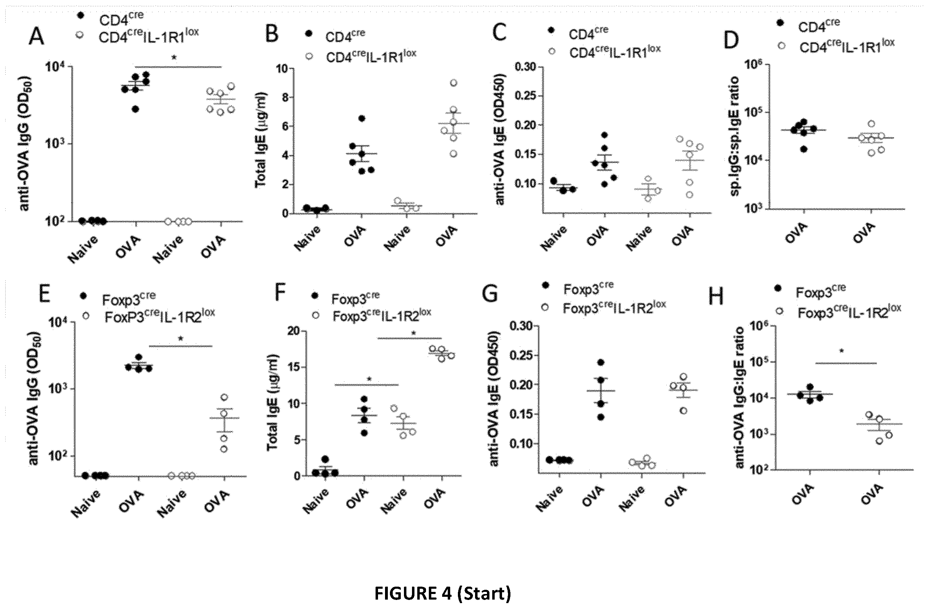

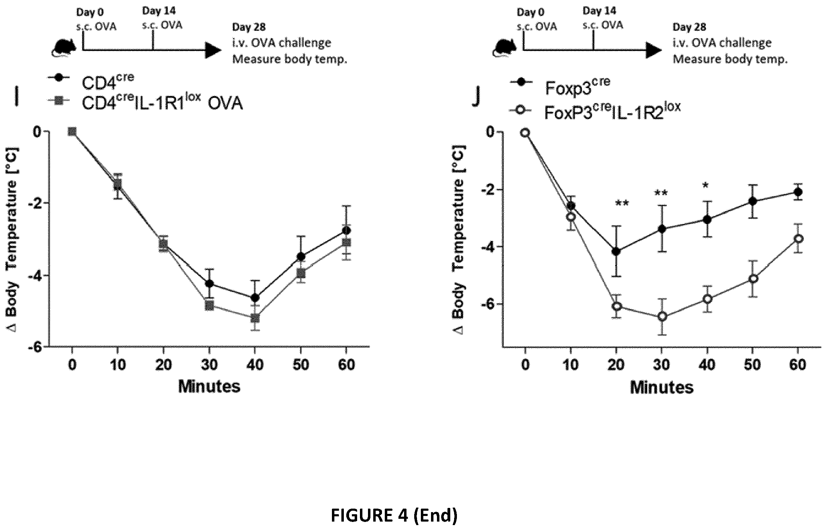

**Figure 4****. A.** CD4^{cre} (WT), or CD4^{cre}IL-1R1^{lox} were immunized s.c. with OVA/Alum at day 0 and day 14 and serum was collected at day 0 and day 21. Shown are mean ± SEM OD50 titers of OVA-specific IgG. **B.** Mean ± SEM ± SEM total IgE levels (ug/ml). **C.** Mean ± SEM OD450 values of OVA-specific IgE. **D.** Mean ± SEM specific IgG to specific IgE ratio. **E.** FoxP3^{cre}(WT) or FoxP3^{cre}IL-1R2^{lox} mice were immunized s.c. with OVA/Alum at day 0 and day 14 and serum was collected at day 0 and day 21. Shown are mean ± SEM OD50 titers of OVA-specific IgG . **F.** Mean ± SEM ± SEM total IgE levels (ug/ml). **G.** Mean ± SEM OD450 values of OVA-specific IgE. **H.** Mean ± SEM specific IgG to specific IgE ratio. **I**. CD4^{cre}IL-1R1^{lox} were challenged i.v with OVA at day 28. Shown are Mean ± SEM degrees lost compared to baseline temperature. **J.** FoxP3^{cre}IL-1R2^{lox} were challenged i.v with OVA at day 28. Shown are Mean ± SEM degrees lost compared to baseline temperature.

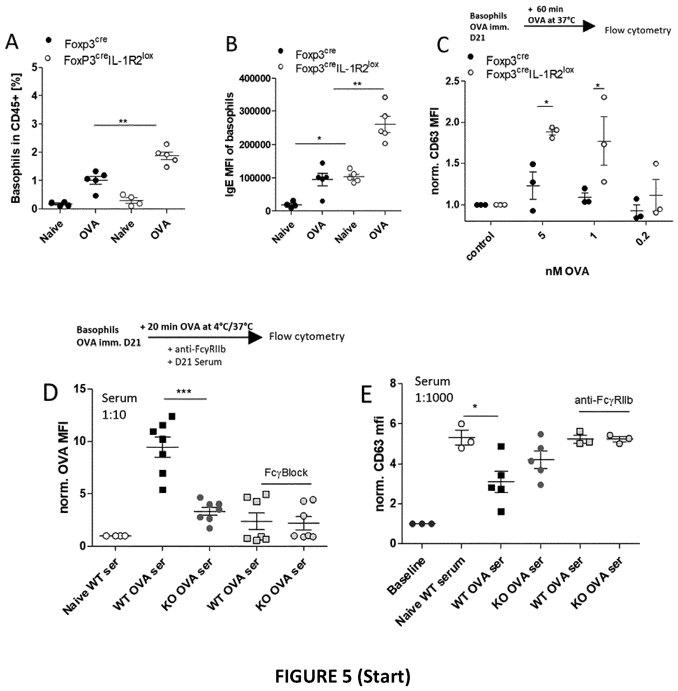

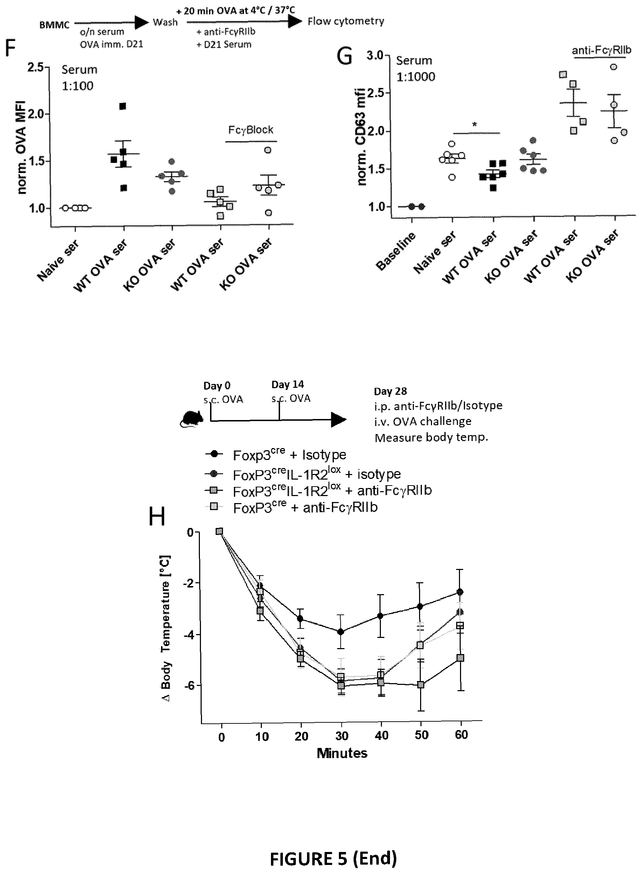

**Figure 5****.** FoxP3^{cre}(WT) or FoxP3^{cre}IL-1R2^{lox} mice were immunized s.c. with OVA/Alum at day 0 and day 14. **A.** Mean ± SEM Basophil frequency at day 21. **B.** Mean ± SEM basophil anti-IgE MFI at day 21. **C.** Mean ± SEM Basophil anti-CD63 MFI upon OVA challenge. **D.** OVA-A488 binding to basophils in presence of 1:10 serum. **E.** Serum inhibition assay with basophils in presence of 1:1000 serum. **F.** OVA-A488 binding to BMMCs in presence of 1:100 serum. **G.** Serum inhibition assay with BMMCs in presence of 1:1000 serum. **H.** 1 hour before OVA challenge at day 28, anti-FcyRllb blocking antibody or isotype control antibody was i.v. injected. Body temperature was measured with a 10-minute interval between each measure. Mean ± SEM degrees lost compared to baseline temperature.

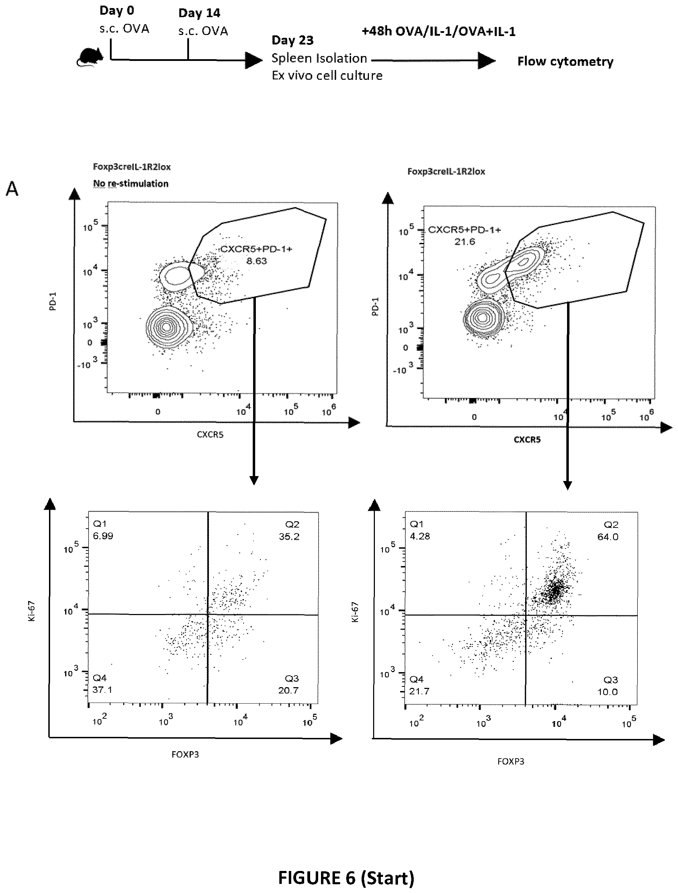

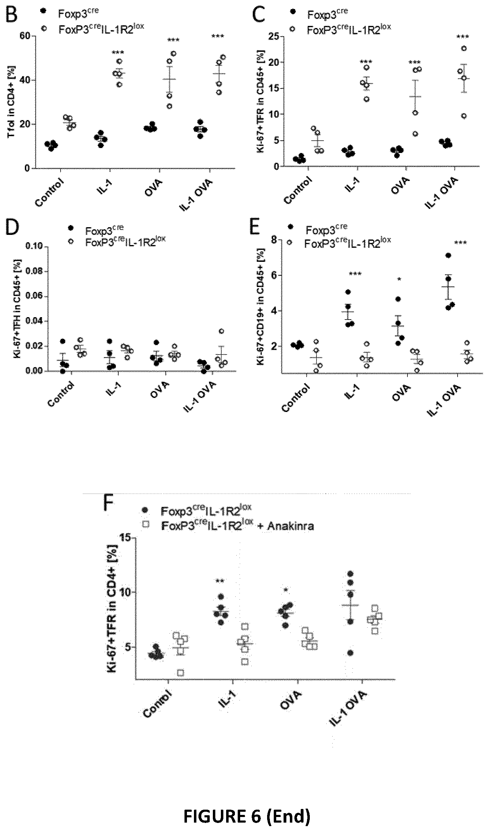

**Figure 6****.** WT or foxp3^{cre}IL-1R2^{lox} mice were immunized s.c. with OVA/Alum at day 0 and day 14. At day 23, spleens were isolated and re-stimulated *ex vivo* with IL-1, OVA or OVA+IL-1β for 48 hours. **A.** Shown is the gating strategy of proliferating CD4+ cells defined as FSH^{hi}KI-67+ in unstimulated cells (left panel) versus IL-1+OVA stimulated cells (right panel) and the expression of PD-1/Foxp3 (lower panels). **B.** Mean ± SEM frequency of total proliferating follicular T cells in CD4+. **C.** Mean ± SEM frequency of proliferating TFR cells in CD45+. **D.** Mean ± SEM frequency of proliferating TFH cells in CD45+. **E.** Mean ± SEM frequency of proliferating CD19+ cells in CD45+. **F.** Anakinra was added to the 48-hour re-stimulation with IL-1, OVA or OVA+IL-1. Shown is the mean ± SEM frequency of proliferating TFR cells in CD4+

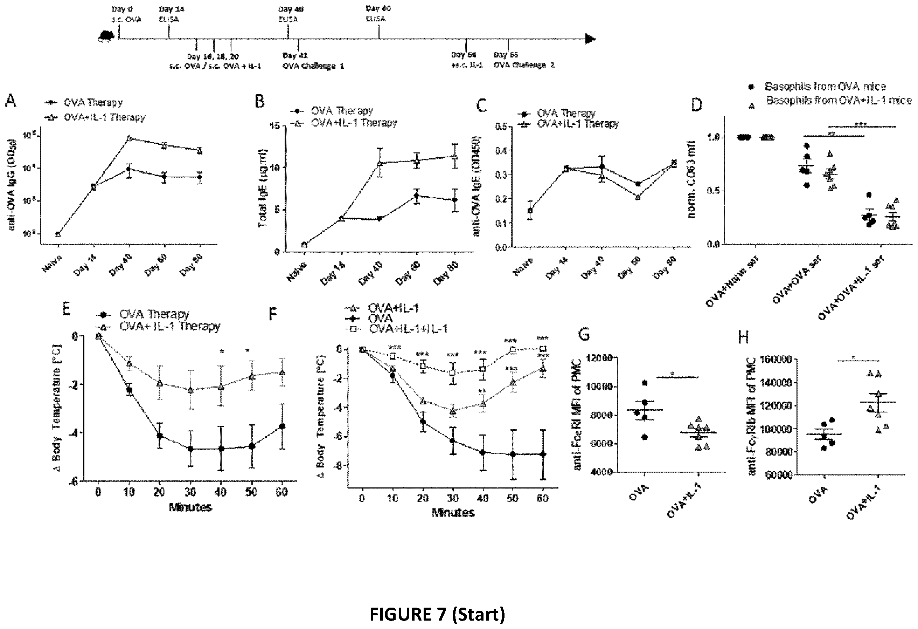

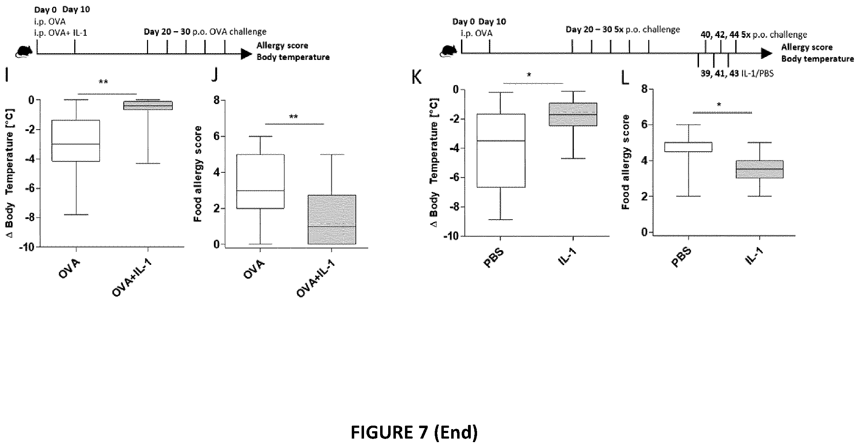

**Figure 7****.** OVA-sensitized mice were treated three times with OVA or OVA+IL-1β in PBS at day 16, 18, 20. Serum was collected before treatment and at day 40. **A.** Shown are mean ± SEM OD50 titers of OVA-specific IgG over time. **B.** Mean ± SEM total IgE levels (µg/ml) over time. **C.** Mean ± SEM OD450 values of OVA-specific IgE over time. **D.** At day 60, Basophils from OVA or OVA+IL-1β treated mice were challenged with OVA in presence of 1:1000 naive, OVA or OVA+IL-1β serum from day 60. Shown is the inhibition of anti- CD63 MFI compared to naive serum. **E.** At day 41, mice were challenged i.v. with OVA. Shown are mean ± SEM degrees lost compared to baseline temperature. **F.** A second challenge was done at day 65 whereas one group had an extra injection of IL-1β in PBS at day 64. Shown are mean ± SEM degrees lost compared to baseline temperature. **G.** At the end of the experiment, PMC were isolated. Shown is the mean ± SEM anti-FcεRI MFI. **H.** Mean ± SEM anti-FcyRllb MFI in PMC. **I**. BALB/c mice were sensitized i.p. at day 0 and day 10 with OVA or OVA+IL-1β before a cycle of p.o. OVA challenges starting at day 20. Shown is the mean ± SEM of maximal temperature drops measured for individual mice. **J.** Shown are mean ± SEM food allergy scores. **K.** At day 40, a second cycle of p.o challenges was performed with addition of s.c. PBS or IL-1β before each challenge. Shown is the mean ± SEM of maximal temperature drops measured for individual mice. **L.** Shown are mean ± SEM food allergy scores.

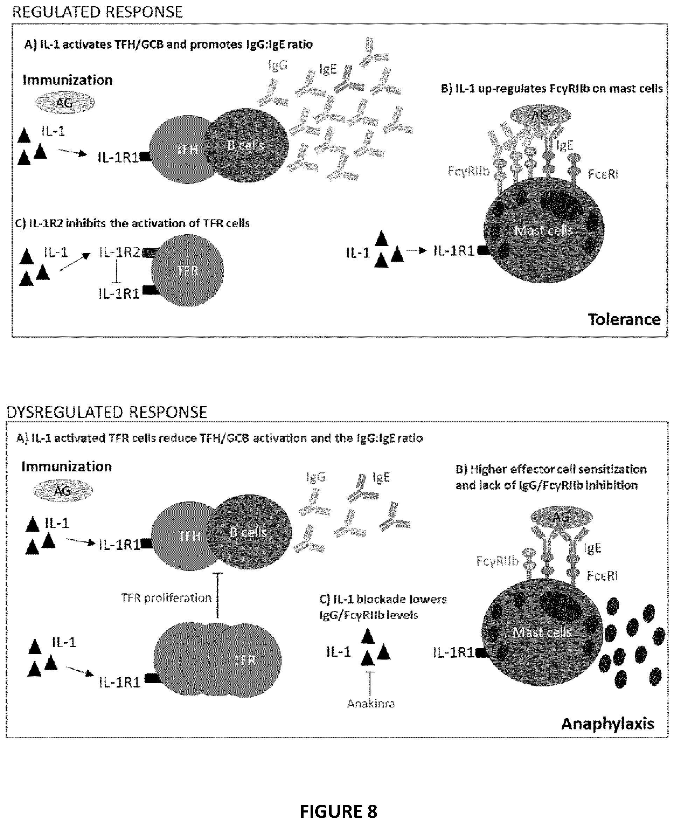

**Figure 8****.** Modes of action of the IL-1 in a regulated response (with tolerance to an allergen) and in a dysregulated response (with anaphylaxis in response to an allergen), as proposed by the Inventors.

### EXAMPLES

### Materials & Methods

**Mice.** All animals were kept at the Centre d'Expérimentation Fonctionnelle animal facility (Paris, France). The local animal ethics committee approved all procedures (agreement number A751315). Transgenic mouse strains were purchased from the Jackson Laboratory (Bar Habor, USA) including B6.129S-IL1rntm1Dih/J (JAX stock #004754), B6.Cg-Tg(Cd4-cre)1Cwi/Bflu/J (JAX stock #022071), B6.129(Cg)-Il1r1tm1.1Rbl/J (JAX stock #028398), B6.129(Cg)-Foxp3tm4(YFP/icre)Ayr/J (JAX stock #016959) except for the strain C57BL/6N-Atm1BrdIl1r2tm1a(EUCOMM)Wtsi/Wtsi (MGI:4842437) that was provided by the Sanger Institute. Seven-week-old BALB/C (AnNR/J) female mice were purchased at Elevage Janvier (Le Genest-Saint-Isle, France). B6.Cg-Tg(Cd4-cre) and B6.129(Cg)-Foxp3tm4(YFP/icre)Ayr/J mice were used as wild type mice. There was no difference of the here-observed IL-1 mediated effects between both strains (not shown). Wild type and transgenic male and female mice were used for experiments between 6 and 15 weeks old. Different groups within individual experiments were performed with mice in the same age ranges of ± 10 days and the same sex.

**Injections.** All injections except OVA challenges were done in a volume of 100ml. Mice were immunized day 0 and day 14 by subcutaneous injection with 100 µg OVA (OVA A5503, Sigma-Aldrich) in Imject^{®} Alum (Thermo Fisher) 1:1 diluted in PBS. Anakinra (Kineret^{®}, Swedish Orphan Biovitrum) was given day 0-5 and day 14-16, twice per day at 50 mg/kg subcutaneously. Mice treated with IL-1β received recombinant mouse IL-1β mixed with OVA/Alum (0.5 µg per mouse; Myltenyi Biotec). For induction of anaphylaxis, mice were injected intravenously with 2 µg OVA in 200ml PBS. Rectal temperature was measured at 10-minute intervals for 1 hour using a rectal thermometer (Biosep Lab Instruments, Vitrolles, France). For blocking of FcγRIIb, 100 ug anti-CD32b antibody (Thermo Fisher, clone AT130-2) were injected intraperitonealy 1 hour before OVA challenge. Alternatively, Mouse IgG2a, kappa Isotype control (Miltenyi Biotec) was injected. For short-term therapeutic injections, 0.5 µg IL-1β in PBS or PBS only was injected subcutaneously. For long-term therapeutic injections 10 µg OVA in PBS or 0.5 µg IL-1β +10 µg OVA in PBS were injected subcutaneously three times with a 2-day interval between each injection. For food allergy sensitization, 10 µg OVA in Alum/PBS 1:1 was injected intraperitoneally at day 0 and day 10. Oral challenge was performed by gavage of 20mg OVA every 2 days for up to 5 challenges, whereas body temperature was assessed at 30-45 minutes. The clinical score was based on the severity of diarrhea graded from 0 to 3 points and appearance of hirsute pelage marked out 0-2 points.

**Sampling.** To assess basophils, blood from tail veins was collected in EDTA tubes (final concentration 1mM EDTA). Red blood cells were lysed using BD Pharm Lyse^{™} (BD Bioscience) according to the manufacturers protocol and were washed three times with PBS. Peritoneal lavage was performed in sacrificed mice by flushing peritoneal cavities with 5ml ice cold PBS under constant massaging. The peritoneum was thereafter cut, and the fluid was collected with a Pasteur Pipet. Red blood cells were lysed followed by three washes with PBS. Whole spleens were collected from sacrificed mice and transferred into 48-well plates containing RPMI 1640 medium (Sigma). The spleens were then mashed and filtered through a cell strainer (70µm, Sigma Aldrich) and washed three times with PBS. To generate murine bone marrow-derived mast cells (BMMCs), femur and tibia of the hind limbs were isolated from sacrificed mice and cut from both sides. The bone marrow was flushed out with a syringe with RPMI 1640 medium. All isolated cells were centrifuged at 300g for 5 min at 4°C and finally suspended in RPMI 1640, 20% FCS medium (Sigma).

**Flow cytometry.** All primary antibody stainings were carried out in 96 round well plates (Thermo Fisher) for 20 minutes at 4°C in PBS. Between all steps, the cells were washed two times with 200µl PBS. Fixation and permeabilization was performed with eBioscience^{™} Intracellular Fixation & Permeabilization Buffer Set (Thermo Fisher) according to the manufacturers protocol. Basophils were defined as anti-mouse CD49b+ (Thermo Fisher, clone DX5), anti-mouse IgE+ (Thermo Fisher, clone 23G3), anti-mouse CD45lo (Thermo Fisher, clone 30-F11) and negative for anti-CD117 (Thermo Fisher, clone 2B8) and anti-CD19 (BD Bioscience, clone 1D3). Peritoneal mast cells (PMC) and bone-marrow derived mast cells (BMMC) were defined as anti-mouse FcεRI+ (Thermo Fisher, clone MAR-1), anti-mouse IgE+, anti-mouse CD117+, anti-mouse F4/80- (Thermo Fisher, clone BM8) and anti-mouse CD49b-. Basophil and mast cell activation was investigated with anti-CD63 (Thermo Fisher, clone NVG-2) staining. Germinal center B cells were defined as CD45+, anti-mouse CD19+ or anti-mouse B220+ (Thermo Fisher, clone RA3-6B2), anti-mouse GL-7+ (Thermo Fisher, clone GL-7) anti-mouse IgD-(Thermo Fisher, clone 11-26c) and anti-mouse CD3- (Thermo Fisher, clone 145-2C11). OVA-specific GCB cells were investigated by staining with 1µg/ml Invitrogen^{™} Ovalbumin, Alexa Fluor^{™} 488 Conjugate (Thermo Fisher Scientific). Proliferating cells were investigated by staining with anti-mouse Ki-67 (Thermo Fisher, clone SoIA15). Plasmablast were defined as CD19+/B220+, GL-7-, IgD-, CD138+ (Thermo Fisher, clone 281-2). Follicular T cells were gated by anti-mouse CD3+, anti-mouse CD4+ (Thermo Fisher, clone RM4-5) anti-mouse CXCR5+ (Thermo Fisher, clone 2G8) and anti-mouse PD-1+ (Thermo Fisher, clone RPM1-30) dumping CD19+ or B220+ cells. In Follicular T cells, TFH cells were defined as anti-mouse FoxP3-(Thermo Fisher, clone FJK-16s) whereas TFR were defined as FoxP3+. All flow cytometry was performed with CytoFLEX (Beckman Coulter, Brea, USA) and analyzed by using FLOWJO software (TreeStar Inc, Ashland, Ore) or CytEXPERT (Beckman Coulter).

**ELISA.** For ELISA, 96-well Nunc Maxisorp ELISA plates (Thermo Fisher Scientific) were coated with reagents in PBS at 4°C overnight. For anti-OVA IgG, 500ng/ml OVA was coated. For total and specific IgE, rat anti-mouse anti-IgE (BD Biosciences, clone R35-72) was coated at 2µg/ml. Blocking was performed with BlockerTM Casein solution (Thermo Fisher) for 2 hours. Dilutions of sera were added to the plates and incubated for 2 hours at room temperature. For specific IgG ELISA, serum was initially diluted 1:100, followed by 10x dilution steps. For total IgE, initial dilution was 1:50 followed by 3x dilution steps. To generate a standard curve, purified mouse IgE (Biolegend, clone MEA-36) was used. For specific IgE, serum dilution was 1:10. The plates were then washed 5 times with PBS/0.05% Tween. Specific IgG was detected for 1 hour at room temperature with biotin rat anti-mouse IgG (Southern Biotech, Birmingham, AL, USA) or biotin rat anti-mouse IgG1 (Southern Biotech), biotin rat anti-mouse IgG2a (Southern Biotech). Total IgE was detected with biotin rat anti mouse IgE (Southern Biotech). After washing 3 times in PBS/0.05% Tween, specific IgG and total IgE ELISAs were further incubated with Streptavidin-HRP (Thermo Fisher) for 1 hour at room temperature. Specific IgE was detected with 500ng/ml OVA added for 1 hour at room temperature in a first step. Plates were washed 5 times with PBS-Tween and thereafter incubated with polyclonal anti-OVA-HRP (Thermo Fisher). Finally, all plates were washed 5 times with PBS/0.05% Tween and the ELISAs were developed with 1x TMB substrate solution (Thermo Fisher) and stopped with 1M HCL. All ODs were measured at 450 nm, the half-maximal antibody titer (OD50) was defined as the reciprocal of the dilution leading to half of the OD measured at saturation.

**Effector cell binding and activation assays.** For basophil assays, about 200ul blood from three mice was pooled and lysed and cells were resuspended in RPMI1640 medium with 10%FBS (Sigma). For basophil activation tests, cells were initially incubated with titrated doses of OVA for 1 hour at 37°C. 5nM OVA was established as a good dose for comparing basophil activation and used for all proceeding experiments. However, the basophil assays were later optimized by shortening the incubation time to 20 minutes at 37°C. For BMMCs, activation was performed for 20 minutes at 37°C using 1nM OVA, based on titrations. Since IgE-antigen complexes do not trigger anaphylaxis the sera was not further purified to minimize the loss of material. The presence of mouse BD Fc-BlockTM (BD Biosciences) further permitted to determine the IgG-dependent binding effect from the sera. For basophil activation tests, serum from D21 immunized mice was pre-mixed for 20 minutes at room temperature with 5nM OVA prior to addition to basophils or 1nM OVA for BMMCs. Binding assays in basophils and BMMCs were performed by premixing 25nM OVA-A488 with 1:10 diluted serum in medium for 20 minutes at 4°C. Activation/Inhibition assays were performed with 1:1000 diluted serum after initial titration experiments. In principle, the same protocol was used for BMMC binding and activation assays except for additional binding experiments using 1:100 serum dilution. For all activation assays, instead of Fcy-block, the more specific blocking antibody anti-FcyRllb was added at 1:200 dilution to the cells prior to the addition of OVA-IgG complexes.

**TFH/TFR re-stimulation assay.** TFH/TFR cells, re-stimulation experiments were performed with whole splenocytes derived from mice immunized at day 21. Isolated splenocytes were cultured at 2 million cells per well in 96 well round (U) bottom plates (Thermo Fisher Scientific). Culture medium was RPMI 1640 (Thermo Fisher Scientific) supplemented with 10% FBS, 20mM L-Glutamine (Thermo Fisher Scientific) and Penicillin-streptomycin (100 U/mL, Thermo Fisher Scientific) in a final volume of 200µl per well. IL-1β, OVA and Anakinra at 0.5ug/ml IL-1β, 1.2 ug/ml OVA and 0.5ug/ml Anakinra were used. The splenocytes were cultured for 48h at 37°C, 5% CO2. The cells were then washed 3 times in PBS prior to staining.

**Statistics.** All results shown are representative of at least 3 independent experiments except for **Figure 2E** (2 independent experiments), **Figure 3A-E** (2 independent experiments). All data is presented as mean plus/minus the standard error of the mean. All the statistical tests and evaluations were performed by using GraphPad PRISM 6.0 (GraphPad Software, Inc, La Jolla, Calif). For all experiments, an α value of 0.05 was used and statistical significance for p-values is displayed as follows: ∗ is less than or equal to .05; ∗∗ is less than or equal to .01; ∗∗∗ is less than or equal to .001. Non-parametric, 2-tailed Student t tests were used for the comparison of two groups (e.g., antibody titers, cell frequencies, activation assays, proliferation assays). Dose- and time-dependent comparisons were performed by 2-way ANOVA followed by Bonferroni correction to obtain individual p-values (e.g., IL-1 titration to BMMC and anaphylaxis experiments).

### Example I - Presence of IL-1 during immunization boosts antibody responses and reduces systemic anaphylaxis

The impact of IL-1 on the allergic immune response was investigated. In a first step, IgG and IgE responses in mice immunized with OVA, OVA+IL-1 or when IL-1 is blocked with a daily Anakinra treatment (OVA+Anakinra) were investigated. Initial antibody serum kinetics permitted to compare day 0 (naïve) and day 21 sera for all conditions (data not shown). **Figure 1A** shows that IL-1β significantly enhanced specific IgG titers whereas IL-1 blockade slightly reduced specific IgG. Similarly, IL-1β led to an increase of total IgE levels **(****Figure 1B****).** Specific IgE levels were very low in general, but an increase in immunized mice compared to naive groups and an enhancement in the OVA+IL-1 groups were observed **(****Figure 1C****).** Basophil-displayed antibodies and serum IgG subclasses were also investigated, all of which were up-regulated in presence of IL-1 (data not shown). Since the IgG/lgE balance is an important characteristic in allergy, IgG:IgE ratio was formulated where a strong increase in OVA+IL-1 immunized mice was observed **(****Figure 1D****).** In a next step, antibody responses upon OVA sensitization in IL-1RaKO mice was investigated. Similar to OVA+IL-1 immunized mice, IL-1RaKO mice displayed higher IgG and IgE responses and a higher IgG/lgE ratio compared to wild type mice **(****Figure 1E-1H****).** Finally, OVA, OVA+IL-1, OVA+Anakinra immunized mice were compared in terms of their anaphylactic response to OVA challenge at day 28. IL-1β protected mice significantly from systemic anaphylaxis while IL-1 blockade had a strong worsening effect **(****Figure 1I****).** Along the same line, IL-1RaKO mice were protected from systemic anaphylaxis compared to wild type mice **(****Figure 1J****).** In conclusion, despite an increase in IgE levels, excess IL-1 during sensitization promotes the IgG:IgE ratio and suppresses the anaphylactic response.

### Example II - IL-1-promoted IgG facilitates the FcγRllb-dependent protection from allergic anaphylaxis

Having shown that IL-1β confers protection against anaphylaxis, the IgG-dependency of this process was investigated. The effect of serum induced by OVA, OVA+IL-1, OVA+Anakinra on the binding of OVA-IgG immune complexes (IC) to whole blood basophils was evaluated (For all titrations and IL-1RaKO serum, data not shown). Alternatively, *in vitro* maturated bone-marrow derived mast cells (BMMC) were used to work with higher cell numbers and purity (phenotypic and functional characterization, data not shown). 1:10 diluted sera (naïve, OVA, OVA+IL-1, OVA+Anakinra) was pre-mixed with fluorescent OVA-A488 to form IC prior to addition to the cells for 30 minutes at 4°C. Since OVA can bind to basophils via IgE in absence of serum, naive serum was used as an OVA binding baseline. **Figure 2A** shows that OVA+IL-1 serum promotes IC binding to basophils compared to naive serum. The additional presence of Fcy-Block completely blocked this enhancement of IC binding. In BMMCs, the effect of OVA+IL-1 serum was even more striking as shown in **Figure 2B****.** In a next step, the effect of the different sera on the degranulation of basophils and mast cells was tested. Titration experiments led to dilute serum 1:1000 for activation assays. Furthermore, a specific anti-FcyRllb blocking antibody was used instead of pan Fcy-Block. OVA+IL-1 serum inhibited basophil and BMMC degranulation more than OVA serum compared to naive serum in FcyRllb-dependent fashion **(****Figure 2C and 2D****).** Finally, mice immunized with OVA, OVA+IL-1, OVA+Anakinra were injected with 100µg anti-FcyRllb antibody or isotype control i.p. 1 hour prior to OVA challenge. OVA or OVA+IL-1 immunized mice injected with the FcyRllb antibody displayed similar levels of enhanced anaphylaxis compared to isotope control antibody injected mice **(Figure 2F).** Hence, the antibodies induced by OVA+IL-1 immunization enhance the Fcy-dependent binding to allergic effector cells and reduces their degranulation in FcyRllb-dependent fashion.

### Example III - IL-1β up-regulates FcyRllb expression in mast cells and reduces their activity

IL-1R1 expression has been previously described on mast cells. Having demonstrated that IL-1β controls allergic anaphylaxis via IgG/FcyRllb, it was evaluated whether IL-1β has a direct effect on the expression of IgE/IgG surface receptors in mast cells. To this end, peritoneal mast cells (PMC) were isolated at day 28 after immunization with OVA, OVA+IL-1 and OVA+Anakinra. Interestingly, it was observed higher frequencies of peritoneal mast cells in OVA+IL-1 immunized mice compared to naive, OVA, and OVA+Anakinra groups **(****Figure 3A****).** However, it was observed decreased FcεRI in OVA+IL-1 immunized mice compared to OVA-immunized mice only **(****Figure 3B****).** Interestingly, the expression of FcyRllb was lower in OVA+Anakinra treated mice compared to OVA and OVA+IL-1 mice **(****Figure 3C****).** Overall the MFI ratios of FcγRIIb:FcεRI, significantly increased in PMCs from OVA+IL-1 immunized mice **(****Figure 3D****).** In a next step, OVA sensitized mice were injected with IL-1β for 1 day before isolating PMC and challenging with OVA. FcyRllb but not FcεRI was up-regulated in PMCs 1 day after IL-1 injection **(****Figure 3E****, 3F).** Mice pre-treated with IL-1 displayed significantly reduced anaphylaxis **(****Figure 3G****).** Mixing IL-1 with Anakinra or the addition of anti-FcyRllb inhibited the protection from anaphylaxis (data not shown). Then, IL-1β to BMMC *in vitro* was titrated for 48 hours and the relative expression of FcεRI and FcyRllb was evaluated by flow cytometry. A significant up-regulation of FcyRllb was detected in IL-1β treated BMMCs whereas FcεRI expression remained more stable **(****Figure 3H, Figure 3I****,).** Then, a serum inhibition assay was performed by adding serum from OVA immunize mice (day 21) to BMMCs primed with IL-1β for 48h. As shown in **Figure 3J****,** IL-1β priming reduced the activity of BMMC in presence of OVA serum compared to naïve serum. Hence, in addition to increasing the IgG:IgE antibody ratio, IL-1β promotes the FcγRIIb:FcεRI receptor ratio and thus reduces the activity of mast cells. In conclusion, the presence of IL-1β during immunization has a dual effect on serum IgG and mast cell FcyRllb that contributes to protection from allergic anaphylaxis.

### Example IV - FoxP3crelL-1R2lox mice display elevated IgE levels, reduced IgG levels, and increased anaphylaxis

Having previously shown that IL-1 activates TFH/GCB cells, the role of IL-1 receptors expressed in follicular T cells in the allergic response was evaluated. To evaluate the role of IL-1R1-expressing follicular T cells in the IgG response mediated by IL-1β, WT (CD4cre) mice or CD4creIL-1R1lox were immunized with OVA or OVA+IL-1 and it was investigated IgG/IgE responses and the anaphylactic response to OVA challenge. As shown in **Figure 4A-4D****,** CD4creIL-1R1lox displayed lower IgG responses, slightly enhanced total IgE responses, a trend towards lower specific IgE, and a non-significant tendency towards lowering of the specific IgG to specific IgE ratio. In a next step, the role of TFR-expressed IL-1R2 was evaluated by studying FoxP3crelL-1R2lox mice. FoxP3crelL-1R2lox mice displayed lower IgG responses but significantly higher naive total IgE levels that were further increased upon immunization **(****Figure 4E****, 4F).** Whereas specific IgE levels did not change in FoxP3crelL-1R2lox mice, the specific IgG: specific IgE ratio was drastically lowered **(****Figure 4H****).** When investigating anaphylaxis in CD4creIL-1R1lox, it was not observed a significant difference compared to WT mice **(****Figure 4I****).** In contrast, FoxP3crelL-1R2lox were significantly more susceptible to systemic anaphylaxis upon intravenous OVA challenge **(****Figure 4J****).** In both strains, CD4creIL-1R1lox and FoxP3crelL-1R2lox, the additional presence of IL-1 during OVA immunization promoted IgG levels, though to a lower level than in WT mice. Nevertheless, OVA+IL-1 immunization had a protective effect on anaphylaxis in both strains compared to OVA only immunization (data not shown). Hence, the absence of IL-1R1 in T follicular cells decreases IgG levels but does not shift the IgG/lgE ratio enough to promote anaphylaxis. In contrast, the absence of IL-1R2 in TFR promotes a prone allergic phenotype characterized by a dysregulated, lower IgG/IgE balance and higher susceptibility to allergic anaphylaxis in response to allergen challenge.

### Example V- Elevated IgE levels and reduced FcyRllb engagement promote anaphylaxis in FoxP3creIL-1R2lox mice

Having observed a dysregulated immune response in FoxP3crelL-1R2lox, the mechanism of anaphylaxis was evaluated in further detail. The higher serum total IgE levels in FoxP3crelL-1R2lox mice come in association with higher blood basophil frequencies compared to WT mice **(****Figure 5A****)** as well as increased surface IgE levels **(****Figure 5B****).** In contrast, serum IgG subclasses, and basophil-IgG were lower (data not shown). In a next step, the reactivity of basophil to OVA challenge in absence of serum was investigated. Basophils from FoxP3crelL-1R2lox showed higher degranulation in response to OVA than WT basophils thus suggesting a pure IgE effect **(****Figure 5C****).** To evaluate the impact of serum IgG, it was investigated the effect of D21 serum from WT or FoxP3crelL-1R2lox mice on effector cell binding as well as activation. In line with the previous results with IL-1 or Anakinra, IgG binding and inhibition of activation correlated with specific IgG levels. It was observed a reduction of IgG-OVA binding to basophils in serum derived from OVA-immunized FoxP3crelL-1R2lox mice compared to WT mice **(****Figure 5D****).** In turn, WT OVA serum inhibited basophil degranulation more than FoxP3crelL-1R2lox OVA serum **(****Figure 5E****).** The same effects were observed in BMMCs **(****Figure 5F****, 5G).** In a next step, it was tested the effect of FcyRllb blockade prior to OVA challenge in FoxP3crelL-1R2lox mice compared to WT mice. As shown in **Figure 5H****,** FoxP3crelL-1R2lox did not benefit from FcyRllb-mediated protection to the same extent as WT mice. In conclusion, the higher IgE levels in addition to a lower IgG:IgE ratio in FoxP3crelL-1R2lox mice leads to higher IgE+ basophil frequencies as well as a lack of FcyRllb engagement on basophils and mast cells resulting in increased allergic anaphylaxis.

### Example VI - FoxP3creIL-1R2lox mice display TFR proliferation in response to IL-1 or antigen re-stimulation

Having shown the mechanism of anaphylaxis in FoxP3crelL-1R2lox mice, it was investigated how a lack of IL-1R2 on TFR results in a shift of the IgG/lgE balance. When comparing splenic frequencies of TFH/TFR/GCB in WT and FoxP3crelL-1R2lox mice, it was observed a striking reduction of GCB cells, higher levels of proliferating TFR and increased B cell apoptosis in FoxP3crelL-1R2lox mice (data not shown). Thus, an *in vitro* re-stimulation experiment was performed to evaluate the effect of IL-1, OVA, or IL-1+OVA re-stimulation on the proliferation of TFR, TFH and B cells. The cells were cultured *ex vivo* for 48 hours in presence of OVA and/or IL-1β. Proliferating cells as Ki-67+FSChi were characterized by flow cytometry (gating shown in **Figure 6A****).** Compared to splenocytes derived from WT mice, follicular T cells in FoxP3crelL-1R2lox displayed a significant proliferative response to IL-1, OVA and IL-1+OVA re-stimulation **(****Figure 6B****).** The proliferating cells were mainly FoxP3+ whereas no proliferation was observed in the FoxP3- cell population **(****Figure 6C****, 6D).** The enhanced TFR proliferation led to an increase in TFR CD69 and ICOS expression, and an increase in the TFR:TFH ratio (data not shown). In contrast, proliferation of B cells was only observed in splenocytes derived from WT mice whereas B cell proliferation was completely suppressed in FoxP3crelL-1R2lox **(****Figure 6E****).** To confirm the IL-1-dependency of TFR proliferation, an assay with FoxP3crelL-1R2lox splenocytes was performed in absence or presence of Anakinra. As shown in **Figure 6H,** TFR proliferation was blocked by the addition of Anakinra, most notably also in OVA only re-stimulated splenocytes suggesting that endogenous IL-1 is in the system. Together these results show that mice lacking IL-1R2 in FoxP3+ cells present higher levels of TFR proliferation and reduced B cell proliferation in response to IL-1β and/or antigen re-stimulation. This dysregulated response may disrupt the TFH/GCB response, lower the IgG:IgE balance and lead to increased allergic responses.

### Example VII - IL-1β protects sensitized mice from systemic and oral allergen challenge

Having observed that IL-1 regulates both mast cells and follicular T cells to suppress allergic responses, it was evaluated whether three subcutaneous IL-1+OVA injections diluted in PBS have a desensitization effect in OVA sensitized mice. It was investigated serum antibody levels before the treatment at day 14, and after the treatment at days 40, 60, and 80. In line with the previous results, IL-1+OVA immunotherapy boosted specific IgG and total IgE levels significantly whereas no changes in specific IgE levels were observed **(****Figure 7A-7C****).** At day 80, a basophil activation test was performed in presence of serum. Basophils from OVA or OVA+IL-1 groups were compared in presence of 1:1000 naïve, OVA or OVA+IL-1 serum (from day 80). There was no difference in basophil activation between OVA or OVA+IL-1 groups, but OVA+IL-1 serum significantly reduced basophil activation **(****Figure 7D****).** A first challenge was performed at Day 41, where IL-1+OVA treated mice were protected from systemic anaphylaxis compared to OVA mice **(****Figure 7E****).** One day before the second challenge at day 65, one group received an extra injection of IL-1 in PBS. **Figure 7F** shows, that IL-1+OVA mice at day 65 were still significantly protected compared to OVA mice, and the extra addition of IL-1 had an additive effect leading to complete absence of anaphylaxis. At the end of the experiment at day 80, PMC were isolated. **Figure 7G** shows that FcεRI expression was reduced whereas FcyRllb expression increased by IL-1+OVAtherapy. Finally, the therapeutic potential of IL-1 was confirmed in a second model of allergy. For this model, BALB/C mice were used, and challenged orally by OVA gavage. In accordance with our model in C57BL/6 mice, the presence of IL-1 during sensitization had a preventive anti-allergic effect, reducing the loss of body temperature and the allergy score **(****Figure 7I and 7J****).** Along the same lines, IL-1 treatment of sensitized mice between OVA challenges reduced anaphylaxis and allergy scores in response **(****Figure 7K and 7L****).** Hence, it was demonstrated the short-term and long-term therapeutic potential of IL-1 in a variety of applications, different mouse strains and different types of allergen challenge.

The mechanisms of action of IL-1 as proposed by the Inventors are summarized in **Figure 8****.**

## Claims

1. Interleukin-1 (IL-1) for use in the prevention and/or the treatment of an allergy.

2. Composition comprising IL-1 as an active ingredient for use in the prevention and/or the treatment of an allergy.

3. Composition for its use according to claim 2, said composition being used in a method of desensitization of an allergic individual.

4. Composition for its use according to claim 2 or 3, said composition being used in a method of immunotherapy.

5. Composition for its use according to any one of claims 2 to 4, said IL-1 being used in combination with at least an allergen.

6. Composition for its use according to any one of claims 2 to 5, wherein said composition further comprises at least an allergen.

7. Composition for its use according to any one of claims 2 to 6, wherein said allergen is a food, contact or respiratory allergen or a drug.

8. Composition for its use according to any one of claims 2 to 7, said composition being administered by oral route, epicutaneous route, subcutaneous route, transdermal route, intralymphatic route, intramuscular route, intravenous route, nasal route, or rectal route.

9. Composition comprising IL-1 and at least an allergen.

10. Composition according to claim 9, said composition being in the form of a solution, a gel, a powder, an aerosol spray, a lotion, or a foam.

11. Medical device comprising the composition according to claim 9 or 10, said medical device being appropriate for the administration of said composition to an individual.

12. Medical device according to claim 11, said medical device being selected from the group comprising a patch, an inhaler, a nebulizer, and a syringe.

13. Kit for desensitization comprising:
- a composition comprising IL-1, and
- at least an allergen.
